# EUROPEAN PATENT APPLICATION

(11) **EP 1 637 103 A1**
(43) Date of publication of application: **22.03.2006**
(21) Application number: 04746013.4
(22) Date of filing: 10.06.2004
(51) Int. Cl.: A61F 13/496

(54) **PLAY-IN-WATER TRUNKS TYPE DIAPER**

(30) Priority: 13.06.2003 JP 2003169775; 29.03.2004 JP 2004095232
(71) Applicant: DAIO PAPER CORPORATION, Iyomishima-shi, Ehime 799-0492 (JP)
(72) Inventor: SEIKE, Yumiko, Shikokuchuo-City, Ehime 7990431 (JP); ITO, Kazunori, Shikokuchuo-City, Ehime 7990431 (JP)
(74) Representative: Hooiveld, Arjen Jan Winfried
(86) International application number: PCT/JP2004/008485
(87) International publication number: WO 2004/110327

(57) **Abstract**

A disposable pants-type diaper for playing in the water is designed to prevent a wearer's excretory substance, super absorbent polymer (SAP), pulp or the like included in an absorber composing an absorbable main body from flowing out of openings or the like of the pants, a wearer of the diaper can move freely, and the diaper does not slip down in the water. The absorbable main body is formed at least by a liquid permeable top sheet, a liquid impermeable sheet and the absorber intervening between the sheets, and three-dimensional gathers which stand upright on the side of the top sheet of the absorbable main body form an excretory substance housing pocket. Further, an elastic stretch member is disposed on a waistband portion of the absorbable main body and side portions between the waistband portion and leg portions.

## Description

### TECHNICAL FIELD

The present invention relates to a pants-type diaper for playing in the water, and more concretely relates to a disposable pats type diaper for playing in the water that is suitable for swimming pants which are worn by children, such as babies in particular, at the time of playing in the water, swimming and the like, and for underpants which are worn under swimming pants.

### BACKGROUND ART

Normally, disposable diapers such as pants-type paper diapers are constituted at least by providing three-dimensional gathers for preventing liquid leakage onto an upper surface of a pants-type absorbable main body formed with a liquid permeable top sheet, a liquid impermeable sheet and an absorber intervening between said sheets, namely, an upper surface of the top sheet, and by bonding the absorbable main body onto an outer sheet of a pants-type diaper formed with non-woven fabric or the like, namely, on a use surface of a back sheet. In order for the absorber to absorb excretory substances such as urine and stool which are evacuated onto the upper surface of the top sheet, the absorber mainly contains a combination of pulpwood such as fluffy woodpulp or fluffy pulp, and super absorbent polymer (abbreviated as "SAP") (for example, see Japanese Patent Application Laid-Open No. 10-314217).

Meanwhile, conventionally, swimming pants for children to be used for playing in the water and swimming come onto the market and are used.

When children wear such disposable paper diapers, however, since the absorbers have thickness, groin portions become bulky and the motions of leg portions are restrained. Further, when children such as babies wear those diapers and play and swim in paddling pool at home or in public pools such as swimming schools for babies, the SAPs included in the absorbers absorb water and the weight of the paper diapers increases, thereby causing a problem that a degree of freedom is further lost. Also, when the diapers are attached and fixed to babies only by elastic stretch members provided to waistband portions, the diapers easily slip down because water resistance is further applied to the diapers whose weight increases in the water. Furthermore, when the SAPs and the pulpwood flow out of openings of the waistband portions or the like and the diapers slip down in the water, an excretory substance leakage preventing function is lost, so that the excretory substances leak out into the water. For this reason, such diapers cause the sanitary problems, and thus are not preferable for playing in the water.

On the other hand, since conventional swimming pants for children are not provided with any excretory substance leakage preventing function, when children such as babies who wear the swimming pants evacuate excretory substances such as urine and stool, the excretory substance immediately flows out of the openings or the like of the pants and contaminate the water in pools or the like, thereby causing more serious sanitary problem.

Conventionally, paper diapers which do not use SAPs are publicly known, but such paper diapers are only used for wearer's training, and thus they are not regularly worn by children, and further are not used for playing in the water. For this reason, they do not solve the above problem.

The present invention is devised in view of the above circumstances, and its object is to provide a disposable pants-type diaper for playing in the water with which excretory substances of wearers, SAP, pulpwood or the like included in an absorber composing an absorbable main body do not flow out of openings or the like of the pants, the wearers can move freely and the diaper does not slip down in the water.

### DISCLOSURE OF THE INVENTION

The object of the present invention is attained by providing a pants-type diaper for playing in the water, characterized in that an absorbable main body is formed by a liquid permeable top sheet, a liquid impermeable sheet and an absorber intervening between the sheets, and three-dimensional gathers, which stand upright on the side of the liquid permeable top sheet on both side ends of the absorbable main body, forming an excretory substance housing pocket on the liquid permeable top sheet.

Further, the object of the present invention is attained more effectively by providing a pants-type diaper for playing in the water, characterized in that an elastic stretch member is disposed at a waistband portion on the absorbable main body and side portions between the waistband portion and leg surrounding portions.

The object of the present invention is attained more effectively by providing a pants-type diaper for playing in the water, characterized in that in the elastic stretch members on the side portions, a tightening force per width of 1 cm at the time of attachment is 140 gf/cm or more.

The object of the present invention is more effectively attained by providing a pants-type diaper for playing in the water, characterized in that a liquid permeable back sheet made of non-woven fabric is disposed on an outermost surface of the absorbable main body.

The object of the present invention is attained more effectively by providing a pants-type diaper for playing in the water, characterized in that water pressure resistance of the non-woven fabric of the back sheet is not less than 100 mmH₂O and not more than 500 mmH₂O.

The object of the present invention is attained more effectively by providing a pants-type diaper for playing in the water, characterized in that the three-dimensional gathers are constituted so as to stand upright according to slipping-down of the diaper.

Further, the object of the present invention is attained more effectively by providing a pants-type diaper for playing in the water, characterized in that a volume of the excretory substance housing pocket formed on the upper surface of the top sheet is 600 to 800 cm³.

The object of the present invention is attained more effectively by providing a pants-type diaper for playing in the water, characterized in that an amount of the pulpwood included in the absorber is not more than 10 g.

The object of the present invention is attained more effectively by providing a pants-type diaper for playing in the water, characterized in that the absorber does not include pulpwood.

The object of the present invention is attained more effectively by providing a pants-type diaper for playing in the water, characterized in that an amount of super absorbent polymer (SAP) included in the absorber is not more than 10 wt%.

The object of the present invention is attained more effectively by providing a pants-type diaper for playing in the water, characterized in that the absorber does not include super absorbent polymer (SAP).

Further, the object of the present invention is attained more effectively by providing a pants-type diaper for playing in the water, characterized in that a disposed area of the liquid impermeable sheet is larger than an area of the absorber and is not more than 70% of a flat area of the absorbable main body.

According to the pants-type diaper for playing in the water of the present invention (hereinafter, "the diaper"), since excretory substances are housed and held in the excretory substance housing pocket, they do not flow out of the openings or the like of the pants. Further, since the amount of the pulpwood included in the absorber is small or the absorber does not include the pulpwood, an amount of the pulpwood to flow out of the absorber can be restricted to be extremely small or zero, and thus the water in a pool or the like can be prevented from being contaminated. Furthermore, because the amount of the pulpwood included in the absorber is small or the pulpwood is not included, bulkiness of the product itself is eliminated, and even when the absorber gets wet, it does not absorb moisture much, so that the wearer can move freely in the water. It is preferable that the amount of the pulpwood included in the absorber is not more than 10 g, and this makes the effectiveness more noticeable.

Particularly according to the diaper in which the elastic stretch member is disposed on the waistband portion in the absorbable main body and the side portions between the waistband portion and the leg surrounding portions, since both the waistband portion and the side portions hold the diaper, the diaper can be prevented from slipping down in the water securely. As a result, the free movement can be further secured, and the excretory substances, the pulp and the like can be securely prevented from flowing out of the openings of the pants. It is preferable that a tightening force of the flexible stretch members of the side portions per width of 1 cm at the time of attachment is not less than 140 gf/cm, and thus the effect of preventing the slipping-down of the diaper in the water can be heightened.

Further, the diaper is preferably disposed with a liquid permeable back sheet composed of non-woven fabric on an outermost surface of the absorbable main body, and the water pressure resistance of the non-woven fabric of the back sheet is preferably not less than 100 mmH₂O and not more than 500 mmH20. By satisfying these conditions, the excessive entering of water into the diaper can be prevented while the diaper has the liquid permeability.

The diaper is preferably designed to have the three-dimensional gather which stands upright according to the slipping-down of the diaper, and, with this design, even when the diaper slips down, the three-dimensional gathers can be always maintained in a state where it is firmly attached to the wearer's skin, thereby securely preventing the excretory substances from flowing out of the excretory substance housing pocket. It is preferable that the volume of the excretory substance housing pocket is 600 to 800 cm³, and if the volume is set within the range, a large volume of excretory substances can be housed.

Further, the amount of SAP included in the absorber of the diaper is preferably not more than 10 wt% and further the absorber does not include SAP preferably, and thus the flowing-out of SAP in the water can be restricted to be very small or zero. Since SAP does not absorb moisture and the weight of the diaper itself does not increase, the wearer can move freely in the water.

Furthermore, the disposed area of the liquid impermeable sheet of the diaper is preferably larger than the area of the absorber and is preferably not more than 70% of the flat area of the absorbable main body. Such a liquid impermeable sheet prevents the water from strongly bumping against the outside of the absorber and damaging it, a protecting function of the absorber is made to be efficient, and air permeability of the entire diaper can be secured.

### BRIEF DESCRIPTION OF THE DRAWINGS

Fig. 1 is a partial section plan view viewed from a front side of a developed pants-type paper diaper according to one embodiment of the present invention;
Fig. 2 is a developed perspective view of Fig. 1;
Fig. 3 is a front view in which the pants-type paper diaper is completed;
Fig. 4 is a view in the direction of the arrow X-X in Fig. 1;
Fig. 5 is a perspective view in which the pants-type paper diaper according to another embodiment of the present invention is developed;
Fig. 6 is a perspective view in which the pants-type paper diameter is completed;
Fig. 7 is a behavior explanation diagram at the time when the pants-type paper diaper according to the embodiment of the present invention has absorbed the water; and
Fig. 8 is a behavior explanation diagram at the time when a conventional diaper has absorbed the water.

### BEST MODE FOR CARRYING OUT THE INVENTION

The contents of the present invention are explained below based on embodiments, but the present invention is not necessarily limited to the embodiments and it goes without saying that the present invention can be variously changed without departing from the scope of the claims.

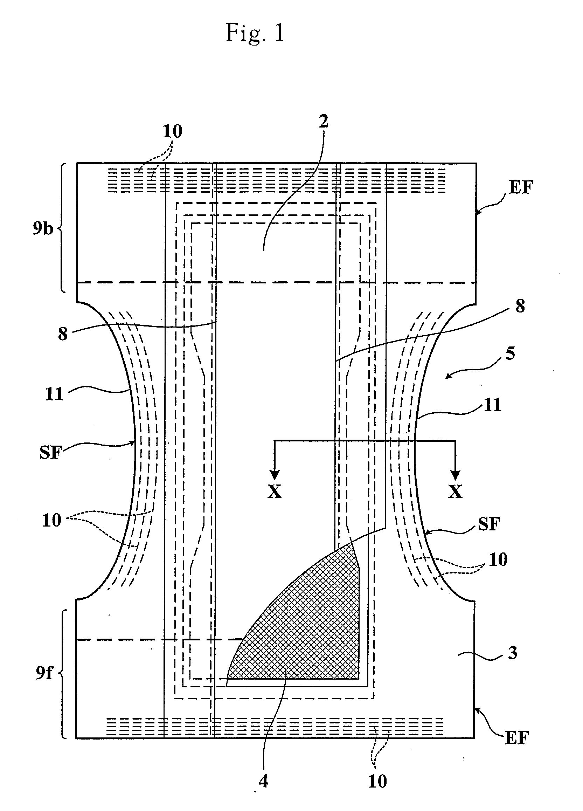

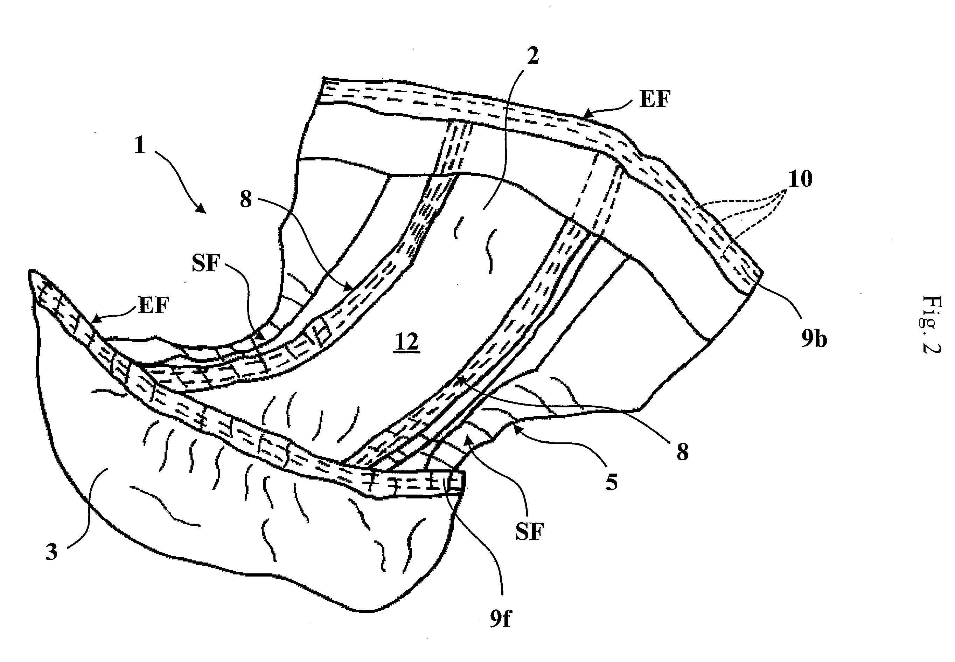

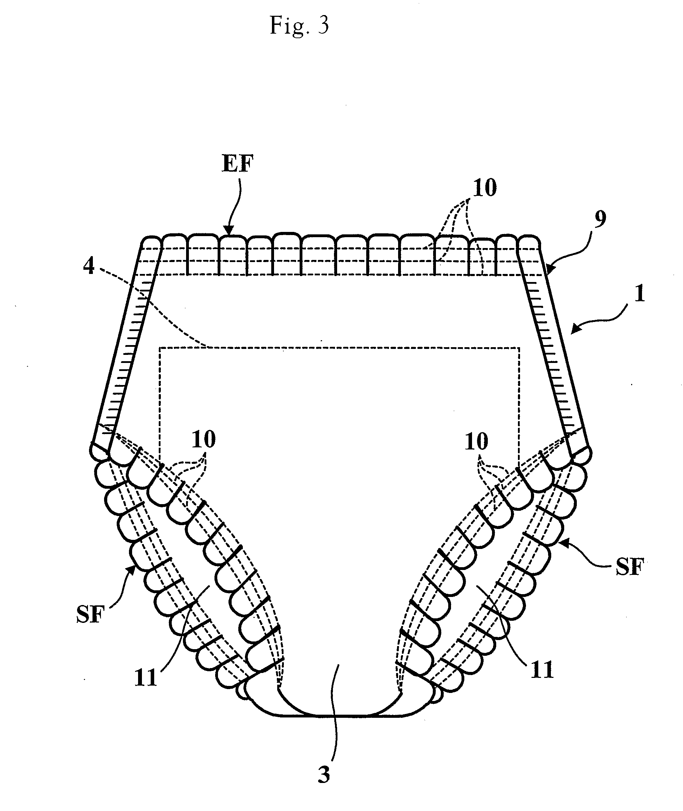

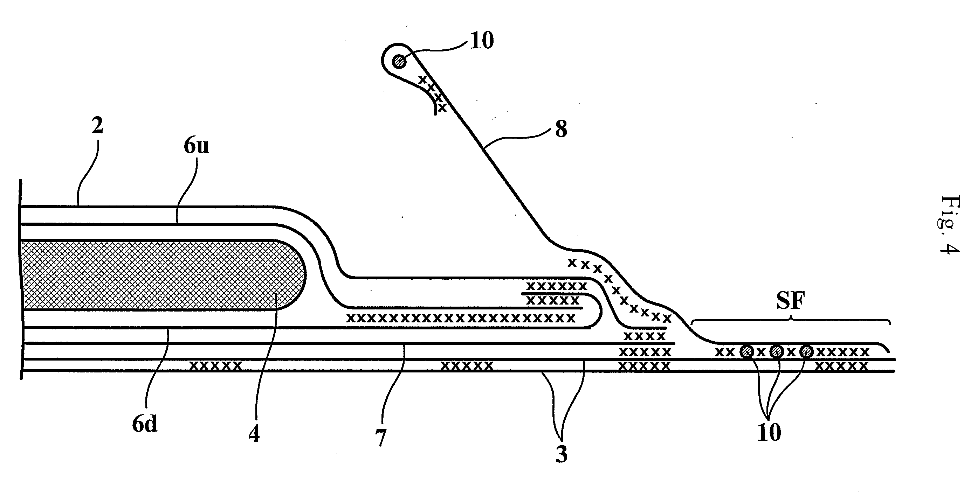

Fig. 1 is a partial sectional plan view viewed from a front side of a developed disposable pants-type diaper 1 for playing in water (hereinafter, "the diaper") according to one embodiment of the present invention. Fig. 2 is a perspective view of Fig. 1, Fig. 3 is a front view in the case where the diaper is formed into a pants shape, and Fig. 4 is a view taken along line X-X in Fig. 1.

The diaper 1 is mainly constituted with an absorbable main body 5 which is mainly formed with a liquid permeable top sheet 2, a liquid impermeable sheet 7 composed of a waterproof film or the like and an absorber 4 which intervenes between the sheets. More specifically as shown in Fig. 4, the absorber 4 is coated with upper creped paper 6u and lower creped paper 6d, a two-layered back sheet 3 composed of non-woven fiber or the like is provided below the liquid impermeable sheet 7, and three-dimensional gathers 8 which prevent excretory substances such as urine and stool from leaking is provided to both ends on the upper surface of the top sheet 2 in a longitudinal direction.

It is preferable that the non-woven fabric of the back sheet 3 has liquid permeability and its water pressure resistance is not less than 100 mmH₂O and not more than 500 mmH₂O. Further, it is preferable that a use area of the liquid impermeable sheet 7 is larger than a flat area of the absorber 4, and is a minimum flat area, for example, 70% or less of the entire flat area of the back sheet 3. Since the other members are generally known well in this field, their details are not explained here. The other members are bonded to each other as shown by marks "×" in Fig. 4 by adhesive such as hot-melt adhesive as already known.

As shown in Fig. 1, the absorbable main body 5 is formed together with the absorber 4, has a plane with an hourglass shape, and a plurality of elastic stretch members 10 made of elastic braids or the like are disposed on a waistband portion 9b on a back side and a waistband portion 9f on an abdomen side of the absorbable main body 5, thereby forming a waistband portion 9 (hereinafter, "end flap portion EF). Similarly, the elastic stretch members 10 are disposed on right and left sides, thereby forming leg surrounding portions 11 (hereinafter, "side flap portions SF"). The absorbable main body 5 thus formed is bonded at the side ends of the waistband portion 9b on the back side and the waist band portion 9f on the abdomen side integrally by adhesive or the like, and finally, as shown in Fig. 3, the pants-type diaper 1 which is not different from a conventional disposable paper diaper in appearance is completed. The diaper 1 is held mainly to the waist portion of a wearer by the end flap portions EF formed by the waistband portion 9b on the back side and the waistband portion 9f on the abdomen side.

As shown in Fig. 4, one end of each three-dimensional gathers 8 are bonded to the side flap portions SF, and one or a plurality of the elastic stretch member(s) 10 is (are) inserted into the other end, namely, a fore end. The three-dimensional gathers 8 are formed so as to stand upright on the upper surface of the absorbable main body 5 and is provided from an outside in a widthwise direction of absorber 4 to a longitudinal direction, namely, to the end flaps EF as shown in Figs. 1 and 2. The elastic stretch member 10 always makes the three-dimensional gather 8 be attached firmly to the skin of the wearer. The three- dimensional gathers 8 and the end flaps EF provided in such a manner form, on a center portion of the absorbable main body 5,an excretory substance housing pocket (hereinafter, "waste pocket") 12 that houses excretory substances such as urine and stool. In the diaper 1, by way of increasing the height of upright installation of the three-dimensional gathers 8, widening the upright installing range, or the like, the volume of the waste pocket 12 is set to 600 to 800 cm³, and is preferably approximately 700 cm³. The volume is several times as large as that of the conventional paper diapers which are generally used, and thus the diaper 1 is designed to house a large volume of excretory substances in the waste pocket 12.

In the diaper 1, the absorber 4 which occupies the main portion of the absorbable main body 5 is constituted such that a content of the pulpwood is approximately half of that of the conventional paper diapers, and it does not contain SAPs at all. In the present invention, the content of the pulpwood is not more than 10 g, or the absorber 4 may not contain it at all. Further, the amount of SAP that is not more than 10 wt% of the entire absorber is applicable. In the diaper 1, since SAP is not used at all and the amount of the pulpwood used is small, or about half of the conventional amount, even when a wearer of the diaper 1 enters into the water of a pool or the like, SAP does not flow out unlike the conventional diapers, and the flowing-out of the pulpwood can be suppressed to nearly zero.

As mentioned above, in the diaper 1, since the amount of the pulpwood to be used for the absorber 4 is small, the product itself has smaller thickness, and thus the wearer does not feel bulkiness at the time of wearing, and can move his/her legs freely. According to an experiment conducted by the inventors, the diaper 1 was put together, and the maximum thickness of this product, namely, the thickness at the approximate center portion of the absorbable main body 5 shown in Fig. 1 was measured. The thickness was 13.64 mm at a load TO of 0.5 g/cm² and was 7.48 mm at a load TM of 50 g/cm², so that it was confirmed that the thickness was small. Further, when a difference between a water retention quantity of the diaper 1, namely, a weight before the diaper 1 is put into a water tank with water and a weight after the diaper 1 is left in the water tank for 1 hour was obtained, the numerical values became 186 g, and thus it is confirmed that the water retention quantity is large.

As detailed above, the diaper 1 of the present invention is not particularly different from the conventional paper diapers in appearance, but the waste pocket 12 is formed on the upper surface of the top sheet 2 of the absorbable main body 5 by the three-dimensional gathers 8, so a large volume of excretory substances can be housed. Simultaneously, the absorber 4 is composed of not more than 10 g of the pulpwood and SAP of not more than 10 wt%; therefore, even when a wearer wearing the diaper 1 plays in the water or swims, the excretory substances are housed in the waste pocket 12, being prevented from flowing out to the outside, and SAP and the pulpwood are securely prevented from flowing out (even if they flows out, the amount is very small). As a result, there is no concern of contaminating the water in a pool or the like. Furthermore, since the thickness of the absorber 4 can be reduced, when a child wears it, the leg portions can be moved freely.

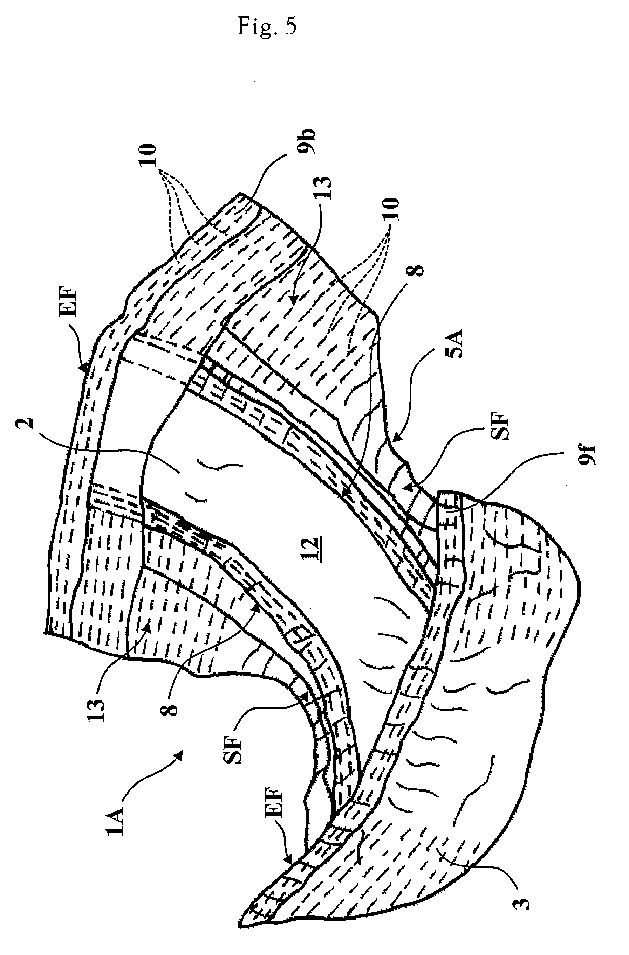

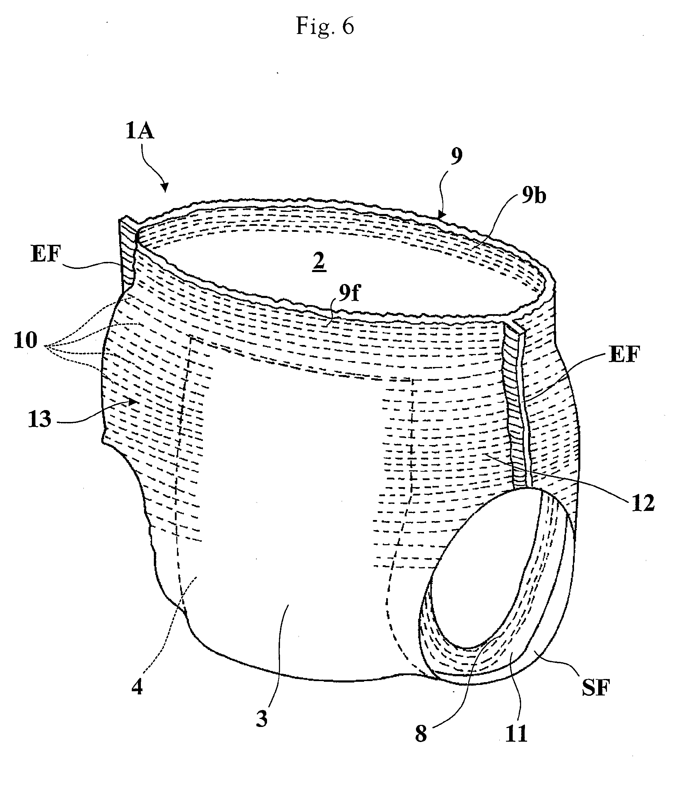

Fig. 5 is a perspective view illustrating a disposable pants-type diaper 1A for playing in the water according to another embodiment of the present invention (hereinafter, "the diaper" as in the above) correspondingly to Fig. 2. Fig. 6 is a perspective view in which the diaper 1A is completed. Since the diaper 1A is substantially the same as the diaper 1 except for a part of the constitution of an absorbable main body 5A, mentioned later, the common components are provided with the same reference numerals.

In the diaper 1A, similarly to the absorbable main body 5, the absorbable main body 5A, formed together with an absorber 4, has a plane in an hourglass shape, and a plurality of elastic stretch members 10 made of elastic braids or the like are disposed on a waistband portion 9b on the back side and a waistband portion 9f on the abdomen side of the absorbable main body 5, thereby forming the end flap portions E. Similarly, the elastic stretch members 10 are disposed on the right and left sides, thereby forming the side flap portions SF. The absorbable main body 5A is, however, different from the absorbable main body 5 in that the elastic stretch members 10 are formed on the end flap portions EF in the absorbable main body 5A and a side portion 13 between the end flap portions EF and the side flap portions SF. According to the experiment by the inventors, it is preferable that a tightening force by means of the elastic stretch members 10 is set to 140 gf/cm or more per width of 1 cm. As the elastic stretch members 10, not only a rubber thread but also, for example, an elastic film can be used.

In the absorbable main body 5A formed in such a manner, the side ends of the waistband portion 9b on the back side and the waistband portion 9f on the abdomen side are bonded integrally by adhesive or the like similarly to the absorbable main body 5. Finally, as shown in Fig. 6, the pants-type diaper 1A which is no different from the conventional disposable paper diapers in appearance is completed.

In the diaper 1A, the elastic stretch members 10 are disposed also on the side portion 13 of the absorbable main body 5A, and the side portion 13 and the waistband portions 9b,9f tighten and hold the diaper 1A at the waist portion through the leg portions of the wearer. For this reason, according to the diaper 1A, the tightening force is increased, and thus the diaper 1A can be prevented from slipping down in the water more securely. As a result, the free movement of the wearer in the water is ensured, and the effect for preventing leakage of excretory substances, pulp and the like is improved.

Further, the diaper 1A is provided with the three-dimensional gathers 8 which always attach firmly to the skin of the wearer similarly to the diaper 1. When the absorber 4 absorbs moisture in the water to increase its weight, the diaper 1 might start slipping down; however, the excretory substances do not leak from the waste pocket 12.

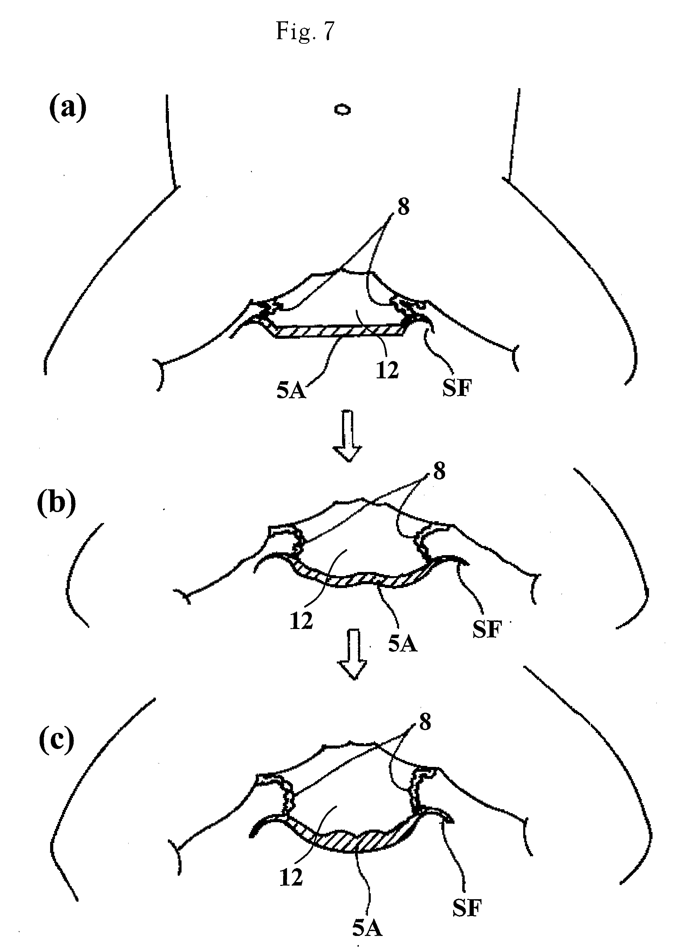

Fig. 7 is a behavior explanatory diagram illustrating that condition. Fig. 7(a) illustrates the case where the absorber 4 does not absorb moisture at all. Fig. 7(b) illustrates the case where the absorber 4 slightly absorbs moisture and the diaper 1A is slipping down slightly. Fig. 7(c) illustrates the case where the absorber 4 absorbs moisture sufficiently and the diaper 1A is slipping down significantly. As shown in the drawings, regardless of the absorbing amount, the three-dimensional gathers 8 always closely contact with the groin portion of the wearer, and a sealed state inside the waste pocket 12 is maintained.

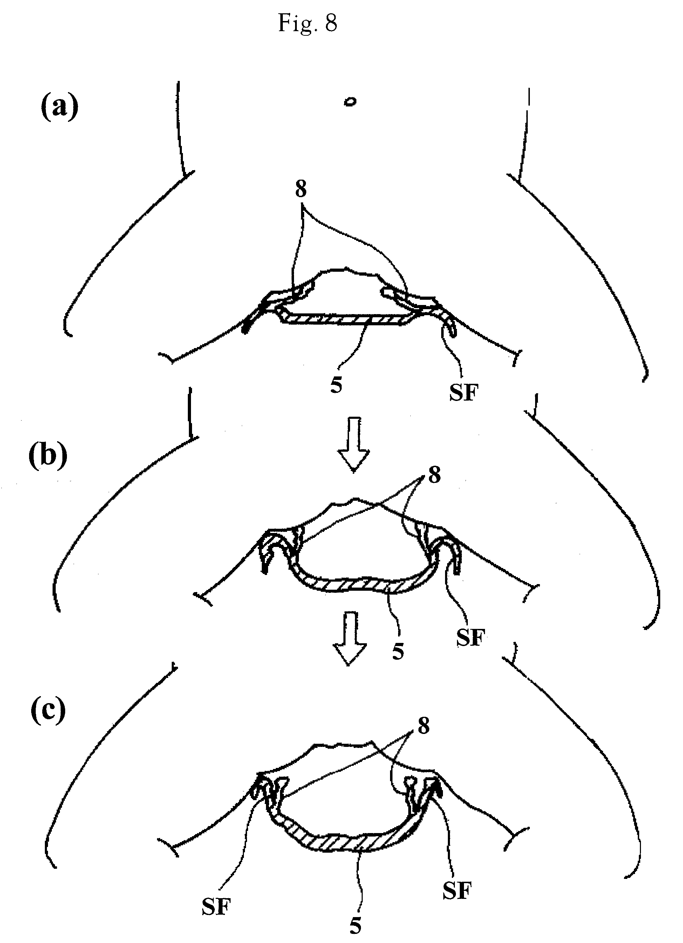

Fig. 8 is a behavior explanation diagram of a conventional diaper shown correspondingly to Fig. 7. That is to say, since the conventional diaper is not provided with the elastic stretch members on the side portions, when it has not absorbed moisture at all, the gap between fore ends of the three-dimensional gathers 8 are narrowed and are attached firmly to the skin in a folded state as shown in Fig. 8(a), and the side flap portions SF surround legs tightly. When the diaper has absorbed a small amount of moisture, the three-dimensional gathers 8 stand upright, whereas the three-dimensional gathers 8 are dragged by the slide flap portions SF losing its function as shown in Fig. 8(b). Further, when the moisture absorbing amount increases and the diaper slips down significantly, the fore ends of the three-dimensional gathers 8 are separated from the wearer's skin as shown in Fig. 8(c), and the function of the waste pocket, namely, the excretory substance housing pocket, is lost, therefore, the excretory substances leak out.

### INDUSTRIAL APPLICABILITY

The pants-type diaper for playing in the water is explained above as the contents of the present invention, but the present invention is not limited to such a diaper for swimming and can be used as a general disposable paper diaper. Further, the water is prevented from being contaminated due to the effluence of wearer's excretory substances, pulp included in the absorber and the like, and the wearer can move freely in the water. For this reason, the present invention can be used also as underpants worn under swimming pants.

## Claims

1. A pants-type diaper for playing in the water, **characterized in that** an absorbable main body is formed with a liquid permeable top sheet, a liquid impermeable sheet and an absorber intervening between the sheets, and three-dimensional gathers, which stand upright on the side of the liquid permeable top sheet on both side ends of the absorbable main body, form an excretory substance housing pocket on the liquid permeable top sheet.

2. The pants-type diaper for playing in the water according to claim 1, **characterized in that** an elastic stretch member is disposed on a waistband portion in the absorbable main body and side portions between the waistband portion and leg portions.

3. The pants-type diaper for playing in the water according to claim 2, **characterized in that** a tightening force of the elastic stretch members on the side portions, per width of 1 cm at the time of attachment is 140 gf/cm or more.

4. The pants-type diaper for playing in the water according to any one of claims 1 to 3, **characterized in that** a liquid permeable back sheet made of non-woven fabric is disposed on an outermost surface of the absorbable main body.

5. The pants-type diaper for playing in the water according to claim 4, **characterized in that** water pressure resistance of the non-woven fabric of the back sheet is 100 mmH₂O or more and 500 mmH₂O or less.

6. The pants-type diaper for playing in the water according to any one of claims 1 to 5, **characterized in that** the three-dimensional gathers are constituted so as to stand upright according to slipping-down of the diaper.

7. The pants-type diaper for playing in the water according to any one of claims 1 to 6, **characterized in that** a volume of the excretory substance housing pocket formed on the upper surface of the top sheet is 600 to 800 cm³.

8. The pants-type diaper for playing in the water according to any one of claims 1 to 7, **characterized in that** an amount of pulpwood included in the absorber is 10 g or less.

9. The pants-type diaper for playing in the water according to any one of claims 1 to 7, **characterized in that** the absorber does not include pulpwood.

10. The pants-type diaper for playing in the water according to any one of claims 1 to 9, **characterized in that** an amount of super absorbent polymer (SAP) included in the absorber is 10 wt% or less.

11. The pants-type diaper for playing in the water according to any one of claims 1 to 9, **characterized in that** the absorber does not include super absorbent polymer (SAP).

12. The pants-type diaper for playing in the water according to any one of claims 1 to 11, **characterized in that** a disposed area of the liquid impermeable sheet is larger than an area of the absorber and is 70% or less of a flat area of the absorbable main body or less.
